# NOUVEAU FASCICULE DE BREVET EUROPEEN

(11) **EP 1 932 118 B2**
(45) Date de publication et mention de la décision concernant l'opposition: **24.08.2016**
(45) Mention de la délivrance du brevet: 05.12.2012
(21) Numéro de dépôt: 06831267.7
(22) Date de dépôt: 02.10.2006
(51) Int. Cl.: G06T 7/00, G06T 7/40

(54) **PROCEDE ET APPAREIL DE CARACTERISATION DES IMPERFECTIONS DE LA PEAU ET PROCEDE D'APPRECIATION DE L'EFFET ANTI-VIEILLISSEMENT D'UN PRODUIT COSMETIQUE**
VERFAHREN UND VORRICHTUNG ZUR BESTIMMUNG VON HAUTMÄNGELN UND VERFAHREN ZUR SCHÄTZUNG DES ANTIALTERUNGSEFFEKTS VON KOSMETIKPRODUKTEN
METHOD AND APPARATUS FOR CHARACTERIZING SKIN BLEMISHES AND METHOD FOR ASSESSING THE ANTI-AGING EFFECT OF A COSMETIC PRODUCT

(30) Priorité: 04.10.2005 FR 0510146
(43) Date de publication de la demande: 18.06.2008
(73) Titulaire: LVMH RECHERCHE, 45800 St. Jean de Braye (FR)
(72) Inventeur: STEPHAN, Sandrine, F-45190 Beaugency (FR); NEVEU, Michèle, F-45000 Orleans (FR); SCHNEBERT, Sylvianne, F-45160 Olivet (FR); PELLE DE QUERAL, Delphine, F-45140 Ingre (FR)
(74) Mandataire: Portal, Gérard
(86) Numéro de dépôt international: PCT/FR2006/050981
(87) Numéro de publication internationale: WO 2007/042708

(56) Documents cités:
- EP-A- 1 314 395
- EP-A- 1 512 372
- EP-A- 1 523 936
- WO-A1-00/76398
- US-A1- 2003 063 801
- US-A1- 2004 122 299
- US-A1- 2004 146 290
- US-A1- 2004 218 810
- US-A1- 2005 119 539
- TOMATIS S. ET AL: 'Image analaysis in the RGB and HS colour planes for a' TUMORI vol. 84, 1998, pages 29 - 32

## Description

L'invention concerne un procédé et appareil de caractérisation des imperfections de la peau et son application dans un procédé de d'appréciation de l'effet anti-vieillissement d'un produit cosmétique.

### ETAT DE LA TECHNIQUE.

Il est connu par le document US 6,551,982 B1 une méthode et un dispositif pour l'estimation non invasive d'un âge relatif d'une personne basés sur une méthode proche de l'infrarouge dite NIR utilisant une longueur d'onde dans le domaine de 700 à 2500 nanomètres.

On connaît encore par le document WO 00/67398 A1 des systèmes et méthodes d'imagerie d'analyse de la peau mettant en oeuvre l'acquisition et la création de diverses images digitales dans le but de visualiser des défauts de peau, et ensuite la prise en compte d'une sous image contenant le défaut de peau.

Il est connu par le document EP 1 523 936 A1 un système permettant l'analyse de la peau d'un sujet utilisant une caméra pour acquérir une image de la peau pour observer les pores et les taches de la peau et utilisant un deuxième appareil autre qu'une caméra pour mesurer une caractéristique physico-chimique de la peau.

### BUTS DE L'INVENTION

La présente invention a pour but de résoudre le problème technique consistant en la fourniture d'un nouveau procédé et d'un nouvel appareil de caractérisation des imperfections de la peau relativement aisés à mettre en oeuvre, sûrs et fiables quant au résultat obtenu.

La présente invention a encore pour but de résoudre le problème technique consistant en la fourniture d'un procédé d'appréciation de l'effet anti-vieillissement d'un produit, cosmétique mettant en oeuvre le procédé ou l'appareil de caractérisation des imperfections de la peau.

La présente invention apporte une solution satisfaisante à ces deux problèmes techniques.

### DESCRIPTION DETAILLEE DE L'INVENTION

Selon un premier aspect, la présente invention fournit un procédé de caractérisation des imperfections de la peau d'une personne tel que défini à la revendication 1.

Selon un mode de réalisation particulier de ce procédé, celui-ci est caractérisé en ce qu'on choisit un plan couleur représentatif de l'imperfection à observer. Par l'expression "représentatif de l'imperfection à observer" qualifiant un plan couleur, on entend un plan couleur permettant d'obtenir visuellement la meilleure mise en évidence de l'imperfection à observer. Sur le plan de couleur choisi, on réalise le paramétrage précité d'au moins un paramètre choisi parmi le niveau de gris moyen de l'image, la surface ou l'aire des imperfections de peau, dont certaines sont liées au vieillissement, et la variance. A titre d'exemple pour l'observation de rides, de ridules et de défauts pigmentaires, on choisira habituellement le plan de couleur bleu, bien que d'autres plans pourraient être également choisis.

Selon un autre mode de réalisation particulier du procédé de l'invention, ladite prise d'image d'au moins une zone de peau est réalisée sous éclairage en lumière blanche ou en lumière ultraviolette. Le choix de l'éclairage entre la lumière blanche et la lumière ultraviolette est dicté par la nature des imperfections à observer. En particulier, l'éclairage en lumière ultraviolette, produit par exemple au moyen d'une lampe de Wood bien connue, permet une bonne mise en évidence des taches pigmentaires et du photo-vieillissement cutané.

Selon un autre mode de réalisation particulier de ce procédé, celui-ci est encore caractérisé en ce qu'on réalise un seuillage des niveaux de gris, c'est-à-dire qu'on élimine les niveaux de gris inférieurs à un certain seuil de niveau de gris prédéterminé, afin d'éliminer des éléments parasites.

Selon encore un autre mode de réalisation particulier de ce procédé, celui-ci est caractérisé en ce qu'on choisit une surface limitée de la peau de la personne à analyser, sur laquelle on procède à l'analyse des imperfections de peau sur la totalité de cette surface.

Selon une variante de réalisation particulière, le procédé est caractérisé en ce que ladite surface limitée de la peau représente une surface d'environ 1 cm² à 10 cm².

Selon une caractéristique avantageuse du procédé sur l'invention, celui-ci est caractérisé en ce qu'on effectue un grossissement de ladite image obtenue par la caméra ou l'appareil photo permettant à un opérateur de mieux visualiser les imperfections de peau et d'évaluer le seuillage des niveaux de gris permettant d'éliminer les éléments parasites ou artéfacts.

Selon un mode de réalisation avantageux du procédé selon l'invention, celui-ci est caractérisé en ce que l'on utilise une caméra couleur numérique de type TRI-CCD ou un appareil photo numérique. Une telle caméra couleur numérique est disponible dans le commerce notamment auprès de la société SONY, et un tel appareil photo numérique, par exemple de type D70S, est disponible dans le commerce auprès de la société NIKON.

Selon un mode de réalisation particulier du procédé sur l'invention, celui-ci est caractérisé en ce qu'on enregistre au moins une image ou une pluralité d'images de la peau d'une même personne, notamment sur plusieurs zones différentes, sur un dispositif d'enregistrement de données numériques.

Selon une variante de réalisation particulière, ladite zone de prise d'image correspond à toute zone cutanée représentative du vieillissement global du visage ou du corps. Avantageusement, il s'agira d'une zone choisie parmi le groupe consistant d'une zone externe de l'oeil (patte d'oie), d'une zone médiane du front, d'une zone de la joue et d'une zone du sillon nasogénien.

Selon un deuxième aspect, la présente invention fournit un appareil de caractérisation des imperfections de la peau d'une personne tel que défini aux revendications d'appareil.

Selon un mode de réalisation particulier de cet appareil, celui-ci est caractérisé en ce que des moyens d'extraction réalisent l'extraction du plan de couleur représentatif de l'imperfection à observer, sur lequel des moyens de calcul réalisent le calcul précité d'au moins un paramètre choisi parmi le niveau de gris moyen de l'image, la surface ou l'aire des imperfections de peau, dont certaines sont liées au vieillissement, et la variance.

Suivant un autre mode de réalisation particulier de l'appareil selon l'invention, celui-ci comprend en outre un dispositif d'éclairage en lumière blanche ou en lumière ultraviolette permettant la prise d'au moins une image d'au moins une zone de peau sous un tel éclairage, en particulier, le dispositif d'éclairage en lumière ultraviolette peut être constitué d'une lampe de Wood.

Selon un autre mode de réalisation particulier de cet appareil, celui-ci est encore caractérisé en ce qu'il comprend des moyens de prise en compte d'un seuillage des niveaux de gris, c'est-à-dire des moyens permettant d'éliminer les niveaux de gris inférieurs à un certain seuil de niveau de gris prédéterminé, afin d'éliminer des éléments parasites.

Selon encore un autre mode de réalisation particulier de cet appareil, celui-ci est caractérisé en ce qu'on prévoit de prendre ladite image d'une surface limitée de la peau de la personne à analyser, sur laquelle des moyens de calcul procèdent à l'analyse des imperfections de peau sur la totalité de cette surface.

Selon une caractéristique avantageuse de l'appareil sur l'invention, celui-ci est caractérisé en ce qu'il comprend des moyens de grossissement de ladite image obtenue par la caméra numérique ou l'appareil photo numérique permettant à un opérateur de mieux visualiser les imperfections de peau et d'évaluer le seuillage des niveaux de gris permettant d'éliminer les éléments parasites ou artéfacts.

Selon une variante de réalisation particulière, cet appareil est caractérisé en ce qu'il comprend des moyens pour extraire d'une image d'une surface de la peau, prise au moyen de ladite caméra ou appareil photo, une zone d'intérêt présentant une surface d'environ 1 à 10 cm²

Selon un mode de réalisation avantageux de l'appareil selon l'invention, celui-ci est caractérisé en ce que l'on prévoit une caméra couleur numérique de type TRI-CCD ou un appareil photo numérique. Une telle caméra couleur est disponible dans le commerce notamment auprès de la société SONY, et un tel appareil photo numérique, par exemple de type D70S, est disponible dans le commerce auprès de la société NIKON.;

Selon un mode de réalisation particulier de l'appareil selon l'invention, celui-ci est caractérisé en ce qu'il comprend des moyens d'enregistrement d'au moins une image ou une pluralité d'images de la peau d'une même personne, notamment sur plusieurs zones différentes, sur un dispositif d'enregistrement de données numériques.

Selon encore un mode de réalisation particulier de l'appareil selon l'invention, celui-ci est caractérisé en ce qu'il comprend un ordinateur combiné à un moniteur comportant un écran, clavier et souris et comprenant un logiciel intégrant l'ensemble des moyens précités incluant :
- les moyens de découpe de l'image numérique en trois plans couleurs : rouge, vert, bleu, dits R, V, B ;
- les moyens d'extraction d'un seul de ces plans, lequel étant le plan le plus représentatif de l'imperfection à observer;
- les moyens de calcul d'au moins l'un des paramètres suivants à partir de ce plan :
   - le niveau de gris moyen de l'image ;
   - la surface ou aire des imperfections de la peau de ladite image, dont certaines sont liées au vieillissement, comme les rides, ridules, taches, cernes, imperfections pigmentaires, zones de relâchement ;
- les moyens de prise en compte d'un seuillage des niveaux de gris ;
- les moyens de grossissement de ladite image ;
- les moyens d'enregistrement d'au moins une image ou une pluralité d'images de la peau.

Selon un mode de relation avantageux de l'invention du procédé et de l'appareil de caractérisation des imperfections de la peau, les moyens de calcul prennent en compte la moyenne de chaque paramètre obtenu sur les différentes images obtenues respectivement avec les différentes zones du visage considérées, afin d'obtenir chaque paramètre moyen global du visage qui est avantageusement enregistré et peut, selon une variante de réalisation, être utilisé, pour chaque personne, pour constituer une courbe d'étalonnage de l'âge de la peau.

Selon un troisième aspect, la présente invention concerne encore un procédé d'appréciation de l'effet anti-vieillissement d'un produit cosmétique, caractérisé en ce qu'on utilise le procédé de caractérisation des imperfections de la peau sur chaque personne d'un groupe de personnes représentatif, tel que défini précédemment ou tel qu'il résulte de la description suivante ; et pour chaque personne :
- on prend au moins une première image numérique avant traitement avec ledit produit cosmétique d'au moins une zone dé peau déterminée pour déterminer les paramètres de référence de la peau avant traitement;
- on prend au moins une deuxième image numérique après une période de temps prédéterminée dudit traitement avec le produit cosmétique et on détermine les mêmes paramètres sur ladite deuxième image;
- on compare les paramètres de la deuxième image de la même zone de peau obtenue après traitement par rapport aux paramètres de la première image de peau avant traitement ;
- on détermine les moyennes de chaque paramètre sur l'ensemble des personnes du groupe ; et
- on conclut à l'efficacité positive de l'effet anti-vieillissement du produit cosmétique considéré lorsqu'au moins l'un des paramètres moyens considérés choisi parmi les niveaux de gris moyen de l'image, la surface ou alors les imperfections et/ou la variance des niveaux de gris a diminué de façon significative par rapport à la moyenne des paramètres de la première image.

Selon un mode de réalisation avantageux de ce procédé, celui-ci est caractérisé en ce que la diminution significative est obtenue avec une probabilité d'erreur inférieure ou égale à 5%.

Selon un autre mode de réalisation avantageux de ce procédé, celui-ci est caractérisé en ce qu'on réalise cet effet anti-vieillissement sur un panel de personnes d'un groupe représentatif d'une catégorie d'âge donné, afin de déterminer le caractère significatif d'efficacité anti-vieillissement du produit cosmétique considéré.

Selon encore un autre mode de réalisation avantageux de ce procédé, celui-ci est caractérisé en ce que la première image et la seconde image sont enregistrées sur chacune des différentes zones caractéristiques du vieillissement de la peau sur le visage et/ou le corps en particulier:
- une zone externe de l'oeil (patte d'oie),
- une zone médiane du front;
- une zone de la joue, et
- une zone du sillon nasogénien.

Grâce aux procédés et à l'appareil selon l'invention, on résout les problèmes techniques précédemment énoncés d'une manière simple, sûre et fiable.

D'autres buts, caractéristiques et avantages de l'invention apparaîtront clairement à la lumière de la description explicative qui va suivre faite en référence à un mode de réalisation actuellement préféré de l'invention.

### DESCRIPTION DES FIGURES

Dans les figures :
- la figure 1 représente un appareil de caractérisation des imperfections de la peau d'une personne, permettant de mettre en oeuvre le procédé de caractérisation des imperfections de la peau précédemment décrit ;
- la figure 2 représente une courbe d'étalonnage de l'âge apparent obtenu avec un échantillon d'un groupe de 70 femmes ayant un âge compris entre 23 et 54 ans ; et
- la figure 3 représente une courbe de l'âge apparent de la peau d'une personne avant traitement (point A) et après traitement avec un produit cosmétique testé(point B).

### Exemple 1

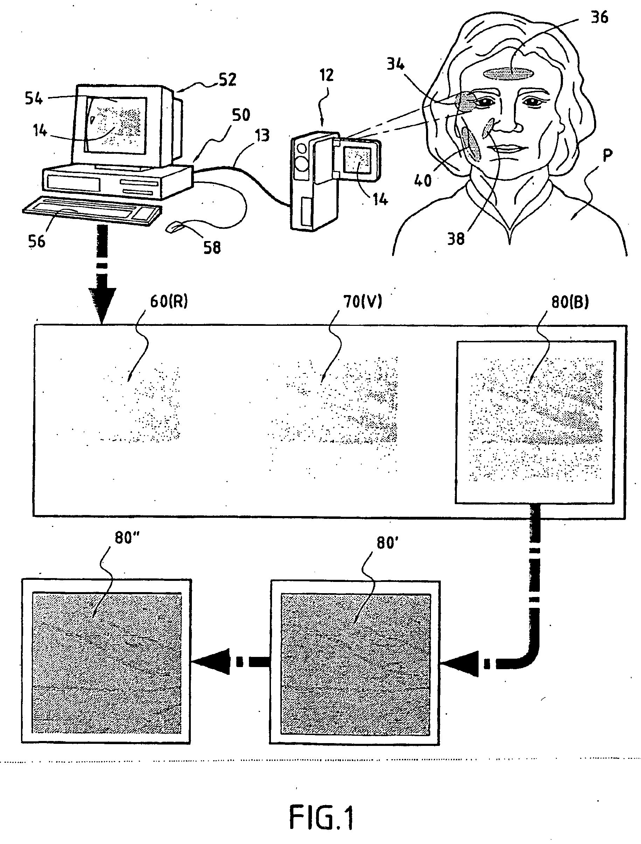

### Description de l'appareil représenté à la figure 1

En référence à la figure 1, on a représenté un mode de réalisation actuellement préféré d'un appareillage selon l'invention, représenté par le numéro de référence générale 10. Pour garantir une précision de positionnement de la personne P, celle-ci sera assise sur une table de repositionnement, par exemple disponible dans le commerce sous la marque VISIOFACE , de la société EOTECH, France.

Cet appareil 10 est prévu pour réaliser la caractérisation des imperfections de la peau d'une personne, et est caractérisé en ce qu'il comprend :
a) une caméra numérique ou appareil photo numérique 12 permettant la prise d'au moins une image numérique 14, d'au moins une zone de peau 34, 36, 38, 40 déterminée, ladite image étant définie par une multiplicité de pixels, que l'on transmet à un dispositif de traitement d'images numériques.

Pour les besoins de cette prise d'images, les zones de la peau déterminées sont éclairées par un dispositif d'éclairage, non représenté, en lumière blanche ou en lumière ultraviolette, selon la nature des imperfections à observer.

Selon un mode de réalisation avantageux de l'appareil selon l'invention, on prévoit une caméra 12 couleur numérique de type TRI-CCD, une telle caméra couleur étant disponible dans le commerce notamment auprès de la société SONY ou un appareil photo numérique de type D70S disponible dans le commerce auprès de la société NIKON Selon une variante de réalisation particulière, ladite caméra ou ledit appareil photo 12 permet de prendre une image d'une surface de la peau ayant environ de 1 cm² à 10 cm².

Egalement, comme cela est bien connu de l'homme de l'art, un tel dispositif de traitement d'images numériques est disponible dans le commerce par exemple sous forme de logiciels, tels que le logiciel d'analyse d'images VISILOG 6.2, que l'on peut intégrer à un ordinateur 50 relié à ladite caméra 12 par un cordon approprié 13. Ledit ordinateur 50 est naturellement combiné à un moniteur 52 avec son écran 54 et son clavier 56 et une souris 58.
b) le dispositif de traitement d'images numériques comprend des moyens de découpe de l'image numérique en trois plans couleurs : rouge 60, vert 70, bleu 80, dits R, V, B,
c) le dispositif de traitement d'images numériques comprend aussi des moyens d'extraction d'un seul de ces plans ; ici de préférence le plan dit bleu 80, correspondant la couleur bleue,
d) des moyens de calcul, ici prévus dans ledit logiciel, d'au moins l'un des paramètres suivants à partir de ce plan :
   - le niveau de gris moyen de l'image ;
   - la surface ou aire des imperfections de la peau de ladite image, dont certaines sont liées au vieillissement, comme les rides, ridules, taches, cernes, imperfections pigmentaires, zones de relâchement ;
   - la variance des niveaux de gris sur l'ensemble des pixels de l'image comme paramètre représentatif de l'homogénéité de la peau.

On notera que le plan bleu 80 est souvent préféré car il présente le plus de contraste et permet de mieux visualiser la plupart des imperfections de peau.

On peut appliquer, sur le plan bleu 80, extrait un filtre mathématique prévu dans le logiciel d'analyse d'images pour éliminer le bruit de parasitage de l'image comme la réflexion.

Selon encore un autre mode de réalisation particulier de cet appareil, celui-ci est caractérisé en ce qu'on prévoit de prendre ladite image d'une surface limitée 34, 36, 38, 40, de la peau de la personne P à analyser, sur laquelle les moyens de calcul procèdent à l'analyse des imperfections de peau sur la totalité de cette surface.

Selon une caractéristique avantageuse de l'appareil sur l'invention, celui-ci comprend des moyens de grossissement, par exemple un grossissement de quatre fois afin d'agrandir les détails, encore intégrés audit logiciel de ladite image obtenue par la caméra permettant à un opérateur de mieux visualiser les imperfections de peau et d'évaluer le seuillage des niveaux de gris permettant d'éliminer les éléments parasites ou artéfacts.

Selon une autre caractéristique avantageuse de l'appareil selon l'invention, celui-ci comprend des moyens de prise en compte d'un seuillage des niveaux de gris, encore intégrés au logiciel, c'est-à-dire des moyens permettant d'éliminer les niveaux de gris inférieurs à un certain seuil de niveau de gris prédéterminé, afin d'éliminer des éléments parasites, en produisant ainsi une image 80' débarrassée des éléments parasites.

On prévoit également avantageusement un deuxième filtrage mathématique, également prévu dans le logiciel, afin d'éliminer les zones minimes détectées lors de l'étape de seuillage précédente et ne présentant pas d'intérêt, en obtenant ici une image 80" sur laquelle les imperfections de peau apparaissent très clairement. C'est sur cette dernière image 80" que les étapes d'analyse d'images et de calcul sont de préférence réalisées.

Ainsi dans le cadre de l'invention, on réalise ces analyses d'images sur les macros-photographies, en particulier 80, 80' ou de préférence 80", de la zone médiane du front 36, sur la zone de la patte d'oie 34, sur la zone du sillon nasogénien 38 et sur la zone médiane de la joue 40.

Selon un mode de réalisation particulier de l'appareil selon l'invention, celui-ci comprend des moyens d'enregistrement, intégrés au logiciel, d'au moins une image ou une pluralité d'images de la peau d'une même personne, notamment sur plusieurs zones différentes ici quatre zones 34, 36, 38 et 40, sur un dispositif d'enregistrement de données numériques intégrées au logiciel.

Selon un autre mode de réalisation particulier de l'appareil selon l'invention, celui-ci est caractérisé en ce que les moyens de calcul prennent en compte la moyenne de chaque paramètre obtenu sur les quatre images obtenues respectivement avec les quatre zones du visage considérées 34, 36, 38 et 40, afin d'obtenir chaque paramètre moyen global du visage qui est avantageusement enregistré et peut, selon une variante de réalisation, être utilisé, pour chaque personne, pour constituer une courbe d'étalonnage de l'âge de la peau.

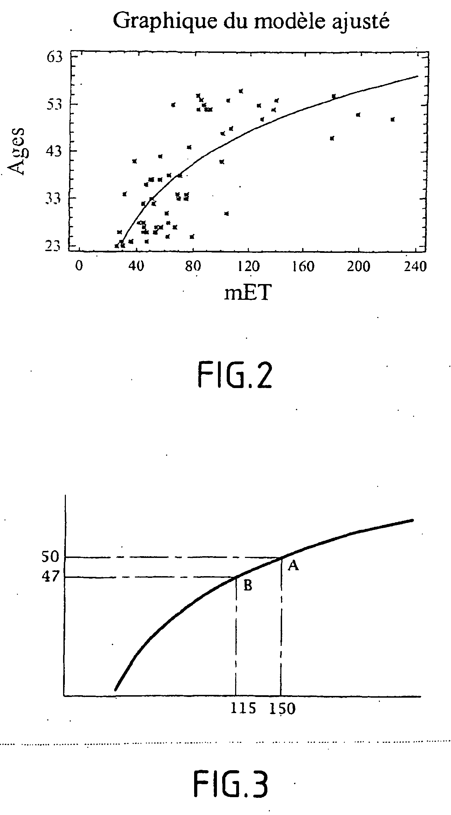

Grâce à l'appareil selon l'invention, on peut mettre en oeuvre l'établissement d'une courbe d'étalonnage de l'âge réel comme indiqué à l'exemple 2, qui permet de déterminer l'âge apparent d'une personne comme montré à la figure 3.

### Exemple 2

### Etablissement d'une courbe d'étalonnage mettant en correspondance l'âge réel avec l'un des trois paramètres de caractérisation des imperfections de la peau choisi parmi le niveau de gris moyen de l'image, la surface ou aire des imperfections de la peau, et/ou la variance des niveaux de gris sur l'ensemble des pixels de l'image

Pour cela, on sélectionne un échantillon de femmes statistiquement représentatif de la catégorie d'âge allant de 20 à 55 ans, constitué de 70 personnes.

Pour chaque personne, on détermine à l'aide du procédé de caractérisation des imperfections de la peau précédemment défini la variance des niveaux de gris sur l'ensemble des pixels de l'image obtenu sur les quatre zones de la peau de chaque personne, constituées par la zone externe de l'oeil (ou patte d'oie), la zone médiane du front ; la zone de la joue et la zone du sillon nasogénien.

On note la variance moyenne obtenue sur ces quatre zones pour chaque personne que l'on reporte sur une courbe orthonormée dont l'abscisse est constituée par la moyenne de la variance des niveaux de gris sur les quatre zones de peau (valeur M), et en ordonnée l'âge réel des personnes du groupe s'étalant de 23 ans à 55 ans, en reportant la totalité des mesures obtenues sur les variances moyennes pour la totalité des femmes. On obtient la courbe représentée à la figure 2, qui est définie par l'équation Ages = -33 + 16.9 x In (M).
In = log népérien dans cette équation.

### Exemple 3

### Test d'évaluation de l'efficacité d'un produit cosmétique

Dans le cadre de cet exemple selon l'invention, on détermine l'efficacité d'un produit cosmétique de composition suivante, en pourcentage en poids :
- Extrait de mauve 3 % (pour ce produit l'extrait de mauve est le produit vitactyl^{®} commercialisé par la société SILAB, Brives, France.
- Extrait de tormentille 0,5 %
- Extrait de bourgeon de hêtre 2 % (produit Gatuline^{®}RC)
- Solution hydro alcoolique d'extraitde *Longosa* contenant 0,5 % en poids sec d'extrait de *Longosa* 2 %
- Gluconate de magnésium 0,05 %
- Excipients émulsionnés sous forme d'huile dans l'eau QSP 100%

Pour réaliser cet essai, on constitue un panel de 30 femmes volontaires d'une catégorie d'âge compris entre 44 et 64 ans, avec une moyenne à 54 ans.

Pour chacune des femmes de ce groupe de 30 personnes, on met en oeuvre la méthode d'appréciation de l'effet anti-vieillissement de ce produit cosmétique, c'est-à-dire que, pour chaque personne :
a) on prend au moins une première image numérique avant traitement dudit produit cosmétique d'au moins une zone de la peau déterminée, ici en pratique de quatre zones de la peau déterminée, à savoir : une zone médiane du front, une zone de la joue, une zone externe de l'oeil (patte d'oie) et une zone du sillon nasogénien.

Ensuite, on détermine la moyenne des données mesurées sur chaque personne pour chaque paramètre comprenant notamment le niveau de gris moyen de l'image, la surface ou aire des imperfections de la peau de ladite première image, dont certaines sont liées au vieillissement, comme les rides, ridules, défauts pigmentaires ; la variance des niveaux de gris sur l'ensemble des pixels de l'image comme paramètre représentatif de l'homogénéité de la peau.

Les moyennes des données mesurées avant le traitement sont rapportées au tableau I.

On procède à l'application du produit cosmétique deux fois par jour, le matin au levé et le soir au couché pendant quatre semaines.

On prend ensuite au moins une deuxième image sur laquelle on réalise les mêmes mesures et déterminations des mêmes paramètres concernant l'aire et la variance qui sont aussi reportées au tableau I avec la mention, "un mois", c'est-à-dire un mois après le début du traitement.

On notera que l'on aurait très bien pu choisir le niveau de gris moyen de l'image comme l'un des paramètres reporté au tableau.

L'appareillage utilisé et la caméra TRI-CCD combinés au logiciel Visilog version 6.2 décrit dans le cadre de l'appareillage de l'exemple 1.

A partir du tableau I, on observe que dans les conditions de l'étude, le produit cosmétique testé améliore significativement l'homogénéité de la peau. On constate en effet une diminution de l'aire des objets détectés sur chaque zone du visage et diminution significative de la variance moyenne sur les quatre zones testées du visage.

**Tableau I**

| **MOYENNE DES DONNEES MESUREES** | | | | |
|---|---|---|---|---|
| **FRONT** | avant | 1 mois | p* | **pourcentage** |
| **aire** | 24987.5 | 20801.1 | S (=0.02) | **-17 %** |
| **variance** | 109.4 | 103.3 | NS | |
| | | | | |

| **OEIL** | avant | 1 mois | p* | **pourcentage** |
|---|---|---|---|---|
| **aire** | 13604.4 | 09786.2 | S (<0.01) | **-28 %** |
| **variance** | 133.5 | 105.7 | S (=0.04) | **-21 %** |

| **SILLON** | avant | 1 mois | p* | **pourcentage** |
|---|---|---|---|---|
| **aire** | 12092.0 | 10546.0 | S (<0.01) | **-13 %** |
| **variance** | 90.1 | 79.7 | S (=0.02) | **-12 %** |
| | | | | |

| **JOUE** | avant | 1 mois | p* | **pourcentage** |
|---|---|---|---|---|
| **aire** | 29830.7 | 22952.2 | S (<0.01) | **-23 %** |
| **variance** | 48.1 | 38.6 | S (<0.01) | **-20 %** |
| | | | | |

| **F+J+O+S** | avant | 1 mois | p* | **pourcentage** |
|---|---|---|---|---|
| **variance** | 95.3 | 81.8 | S (<0.01) | **-14 %** |

On a représenté à la figure 3 une courbe du type de celle obtenue à la figure 2 pour une femme donnée ayant un âge réel de 52 ans.

A partir de la première image, on a mesuré sa variance moyenne sur les quatre zones de peau considérées précitées et on a obtenu le point A qui lui donne un âge apparent de sa peau de 50 ans.

Après traitement avec le produit cosmétique considéré de cet exemple, la variance obtenue donne le point B avec un âge apparent de 47 ans après traitement.

On constate que cette courbe constitue en pratique un indice de mesure de l'effet anti-vieillissement du produit cosmétique qui constitue pour le cosméticien un « indice de jeunesse » pouvant être complété par l'avis d'un expert et l'avis de la femme elle-même sur l'efficacité du produit sur sa peau.

L'invention couvre aussi tous les moyens constituant des équivalents techniques des moyens décrits ainsi que diverses combinaisons.

## Revendications

1. Procédé de caractérisation des imperfections de la peau d'une personne, liées au vieillissement, comprenant l'étape suivante:
a) on prend au moins une image numérique (14) d'au moins une zone de peau déterminée (34, 36, 38, 40) ladite image étant définie par une multiplicité de pixels à l'aide d'une caméra numérique ou un appareil photo numérique (12), que l'on transmet à un dispositif de traitement d'images numériques; **caractérisé en ce qu**'il comprend les étapes suivantes :
b) on choisit une surface limitée de la peau de la personne à analyser, sur laquelle on procède à l'analyse des imperfections de peau sur la totalité de cette surface, on enregistre une pluralité d'images de la peau d'une même personne, sur plusieurs zones différentes, sur un dispositif d'enregistrement de données numériques ; puis on découpe les images numériques ainsi enregistrées en trois plans couleurs : rouge (60), vert (70), bleu (80), dit R, V, B, à l'aide dudit dispositif de traitement d'images ;
c) on extrait un seul de ces plans constitué par le plan de couleur bleue (80), pour l'observation de rides, de ridules et de défaut pigmentaire ;
d) on calcule, par des moyens de calcul appropriés, au moins l'un des parametres suivants a partir de ce plan, constitué par la variance des niveaux de gris sur l'ensemble des pixels de l'image comme paramètre représentatif de l'homogénéité de la peau et les moyens de calcul prennent en compte la moyenne de chaque paramètre obtenu sur les différentes images obtenues respectivement avec les différentes zones du visage considérées, afin d'obtenir chaque paramètre moyen global du visage qui est enregistré et est utilisé, pour chaque personne, pour constituer une courbe d'étalonnage de l'âge de la peau.

2. Procédé selon la revendication 1, **caractérisé en ce que** sur le plan de couleur choisi, on réalise le paramétrage d'au moins un paramètre choisi parmi le niveau de gris moyen de l'image, la surface ou l'aire des imperfections de peau, dont certaines sont liées au vieillissement.

3. Procédé selon l'une des revendications 1 à 2, **caractérisé en ce que** la prise d'image précitée d'au moins une zone de la peau est réalisée sous éclairage en lumière blanche ou en lumière ultraviolette.

4. Procédé selon l'une des revendications 1 à 3, **caractérisé en ce qu'**on réalise un seuillage des niveaux de gris, c'est-à-dire qu'on élimine les niveaux de gris inférieurs à un certain seuil de niveau de gris prédéterminé, afin d'éliminer des éléments parasites.

5. Procédé selon l'une des revendications 1 à 5, **caractérisé en ce que** ladite surface limitée de la peau représente une surface d'environ 1 cm² à 10 cm².

6. Procédé selon l'une des revendications 1 à 5, **caractérisé en ce qu'**on effectue un grossissement de ladite image obtenue par ladite caméra ou ledit appareil photo permettant à un opérateur de mieux visualiser les imperfections de peau et d'évaluer le seuillage des niveaux de gris permettant d'éliminer les éléments parasites ou artéfacts.

7. Procédé selon l'une des revendications précédentes, **caractérisé en ce que** l'on utilise une caméra couleur numérique de type TRI-CCD ou un appareil photo numérique de type D70S.

8. Procédé selon la revendication 8, **caractérisé en ce que** ladite zone de prise d'image correspond à toute zone cutanée représentative du vieillissement global du visage ou du corps ; avantageusement, ladite zone est choisie parmi le groupe consistant d'une zone externe de l'oeil (patte d'oie), d'une zone médiane du front; d'une zone de la joue, et d'une zone du sillon nasogénien.

9. Procédé d'appréciation de l'effet anti-vieillissement d'un produit cosmétique, **caractérisé en ce qu'**on utilise le procédé de caractérisation des imperfections de la peau sur chaque personne d'un groupe de personnes représentatif, tel que défini à l'une quelconque des revendications 1 à 8 ; et pour chaque personne:
- on prend au moins une première image numérique avant traitement avec ledit produit cosmétique d'au moins une zone de peau déterminée pour déterminer les paramètres de référence de la peau avant traitement;
- on prend au moins une deuxième image numérique après une période de temps prédéterminée dudit traitement avec le produit cosmétique et on détermine les mêmes paramètres sur ladite deuxième image;
- on compare les paramètres de la deuxième image de la même zone de peau obtenue après traitement par rapport aux paramètres de la première image de peau avant traitement;
- on détermine les moyennes de chaque paramètre sur l'ensemble des personnes du groupe ;
- on conclut à l'efficacité positive de l'effet anti-vieillissement du produit cosmétique considéré lorsqu'au moins l'un des paramètres moyens considérés choisi parmi la variance des niveaux de gris, a diminué de façon significative par rapport à la moyenne de la variance des niveaux de gris de la première image, et
- on réalise cet effet anti-vieillissement sur un panel de personnes d'un groupe représentatif d'une catégorie d'âge donné, afin de déterminer le caractère significatif d'efficacité anti-vieillissement du produit cosmétique considéré.

10. Procédé selon la revendication 9, **caractérisé en ce que** la diminution significative est obtenue avec une probabilité d'erreur inférieure ou égale à 5%.

11. Procédé selon l'une quelconque des revendications 9 à 10, **caractérisée en ce que** la première image et la seconde image sont enregistrées sur chacune des différentes zones caractéristiques du vieillissement de la peau sur le visage et/ou le corps, en particulier :
- une zone externe de l'oeil (patte d'oie),
- une zone médiane du front;
- une zone de la joue, et
- une zone du sillon nasogénien.

12. Appareil (10) de caractérisation des imperfections de la peau d'une personne liées au vieillissement, comprenant:
a) une caméra numérique ou un appareil photo numérique (12) permettant la prise d'au moins une image numérique (14) d'au moins une zone de peau déterminée (34, 36, 38, 40), ladite image étant définie par une multiplicité de pixels, que l'on transmet à un dispositif de traitement d'images numériques;
**caractérisé en ce qu'**il comprend:
b) des moyens d'enregistrement d'une pluralité d'images de la peau d'une même personne, sur plusieurs zones différentes, sur un dispositif d'enregistrement de données numériques ;
c) des moyens pour extraire de ladite image numérique une surface limitée de la peau de la personne à analyser, sur laquelle des moyens de calcul procèdent à l'analyse des imperfections de peau sur la totalité de cette surface limitée, puis des moyens de découpe de l'image numérique en trois plans couleurs : rouge (60), vert (70), bleu (80), dits R, V, B, à l'aide dudit dispositif de traitement d'images ;
d) des moyens d'extraction d'un seul de ces plans constitué par le plan de couleur bleue (80) pour l'observation de rides, de ridules et de défaut pigmentaire;
e) des moyens de calcul d'au moins l'un des parametres suivants a partir de ce plan constitué par la variance des niveaux de gris sur l'ensemble des pixels de l'image, comme paramètre représentatif de l'homogénéité de la peau ; et les moyens de calcul prennent en compte la moyenne de chaque paramètre obtenu sur les différentes images obtenues respectivement avec les différentes zones du visage considérées, afin d'obtenir chaque paramètre moyen global du visage qui est enregistré et est utilisé, pour chaque personne, pour constituer une courbe d'étalonnage de l'âge de la peau.

13. Appareil selon la revendication 12, **caractérisé en ce que** les moyens d'extraction réalisent l'extraction du plan de couleur représentatif de l'imperfection à observer, sur lequel les moyens de calcul réalisent le calcul précité d'au moins un paramètre choisi parmi le niveau de gris moyen de l'image, la surface ou l'aire des imperfections de peau, dont certaines sont liées au vieillissement.

14. Appareil selon la revendication 12 ou 13, **caractérisé en ce qu'**il comprend en outre un dispositif d'éclairage en lumière blanche ou en lumière ultraviolette permettant la prise d'au moins une image d'au moins une zone de la peau sous un tel éclairage.

15. Appareil selon l'une des revendications 12 à 14, **caractérisé en ce qu'**il comprend des moyens de prise en compte d'un seuillage des niveaux de gris, c'est-à-dire des moyens permettant d'éliminer les niveaux de gris inférieurs à un certain seuil de niveau de gris prédéterminé, afin d'éliminer des éléments parasites.

16. Appareil selon l'une des revendications 15 à 18, **caractérisé en ce que** ladite surface limitée de la peau représente une surface d'environ 1 cm² à 10 cm².

17. Appareil selon l'une des revendications 13 à 16, **caractérisé en ce qu'**il comprend des moyens de grossissement de ladite image obtenue par la caméra (12) permettant à un opérateur de mieux visualiser les imperfections de peau et d'évaluer le seuillage des niveaux de gris permettant d'éliminer les éléments parasites ou artéfacts.

18. Appareil selon l'une des revendications 12 à 17, **caractérisé en ce que** l'on prévoit une caméra (12) couleur numérique de type TRI-CCD ou un appareil photo numérique de type D70S.

19. Appareil selon l'une revendications 12 à 18, **caractérisé en ce que** ladite zone de prise d'image correspond à toute zone cutanée représentative du vieillissement global du visage ou du corps ; avantageusement, ladite zone est choisie parmi le groupe consistant d'une zone externe de l'oeil ou patte d'oie, d'une zone médiane du front, d'une zone de la joue et d'une zone du sillon nasogénien.

20. Appareil selon l'une des revendications 12 à 19, **caractérisé en ce qu'**il comprend un ordinateur (50) combiné à un moniteur (52) comportant un écran (54), un clavier (56) et une souris (58) et comprenant un logiciel intégrant l'ensemble des moyens précités incluant :
- les moyens de découpe de l'image numérique en trois plans couleurs : rouge (60), vert (70), bleu (80), dits R, V, B ;
- les moyens d'extraction d'un seul de ces plans (80) constitué par le plan dit bleu correspondant à la couleur bleue;
- les moyens de calcul d'au moins l'un des paramètres suivants à partir de ce plan :
. le niveau de gris moyen de l'image ;
. la surface ou aire des imperfections de la peau de ladite image, dont certaines sont liées au vieillissement, comme les rides, ridules, taches, cernes, imperfections pigmentaires, zones de relâchement;
- les moyens de prise en compte d'un seuillage des niveaux de gris ;
- les moyens de grossissement de ladite image ;
- les moyens d'enregistrement d'au moins une image ou une pluralité d'images de la peau.

## Patentansprüche

1. Verfahren zur Charakterisierung von mit der Alterung zusammenhängenden Hautunregelmäßigkeiten einer Person, das den folgenden Schritt umfasst:
a) mit Hilfe einer Digitalkamera oder eines digitalen Fotoapparates (12) wird wenigstens ein digitales Bild (14) wenigstens eines bestimmten Hautbereichs (34, 36, 38, 40), wobei das Bild durch eine Vielzahl von Pixeln definiert ist, aufgenommen, das an eine Vorrichtung zur Verarbeitung digitaler Bilder übertragen wird,
**dadurch gekennzeichnet, dass** es die folgenden Schritte umfasst:
b) es wird eine begrenzte Fläche der zu analysierenden Haut der Person gewählt, an der die Analyse der Hautunregelmäßigkeiten über diese gesamte Fläche durchgeführt wird, es wird eine Vielzahl von Bildern der Haut einer gleichen Person, an mehreren unterschiedlichen Bereichen, auf einer Vorrichtung zur Speicherung digitaler Daten aufgezeichnet, anschließend werden die so aufgezeichneten digitalen Bilder mit Hilfe der Bildverarbeitungsvorrichtung in drei Farbebenen, Rot (60), Grün (70), Blau (80), bezeichnet mit R, G, B, zerlegt,
c) es wird eine einzige dieser Ebenen, welche durch die blaue Farbebene (80) gebildet ist, für die Betrachtung von Falten, von Fältchen und einer Pigmentstörung extrahiert,
d) durch geeignete Berechnungsmittel wird wenigstens einer der folgenden Parameter anhand dieser Ebene berechnet, bestehend aus der Varianz der Graustufen über alle Pixel des Bildes als für die Homogenität der Haut repräsentativer Parameter, und die Berechnungsmittel berücksichtigen den Mittelwert eines jeden Parameters, der an den unterschiedlichen Bildern erhalten wird, die jeweils mit den betrachteten unterschiedlichen Bereichen des Gesichts erhalten werden, um jeden durchschnittlichen Gesamtparameter des Gesichts zu erhalten, der gespeichert wird und für jede Person verwendet wird, um eine Eichkurve des Alters der Haut zu bilden.

2. Verfahren nach Anspruch 1, **dadurch gekennzeichnet, dass** auf der gewählten Farbebene die Parametrierung wenigstens eines Parameters, ausgewählt aus der mittleren Graustufe des Bildes, der Oberfläche oder Fläche der Hautunregelmäßigkeiten, von denen einige mit der Alterung zusammenhängen, durchgeführt wird.

3. Verfahren nach einem der Ansprüche 1 bis 2, **dadurch gekennzeichnet, dass** die vorgenannte Aufnahme eines Bildes wenigstens eines Bereiches der Haut unter Beleuchtung mit weißem Licht oder UV-Licht vorgenommen wird.

4. Verfahren nach einem der Ansprüche 1 bis 3, **dadurch gekennzeichnet, dass** ein Graustufen-Schwellwertvergleich durchgeführt wird, das heißt, dass die Graustufen unterhalb eines gewissen vorbestimmten Graustufen-Schwellwerts eliminiert werden, um Störelemente zu beseitigen.

5. Verfahren nach einem der Ansprüche 1 bis 5, **dadurch gekennzeichnet, dass** die begrenzte Fläche der Haut eine Fläche von etwa 1 cm² bis 10 cm² ausmacht.

6. Verfahren nach einem der Ansprüche 1 bis 5, **dadurch gekennzeichnet, dass** eine Vergrößerung des mittels der Kamera oder des Fotoapparates erhaltenen Bildes vorgenommen wird, die einer Bedienungsperson ermöglicht, die Hautunregelmäßigkeiten besser sichtbar zu machen und den Graustufen-Schwellwertvergleich, der ermöglicht, die Störelemente oder Artefakte zu beseitigen, auszuwerten.

7. Verfahren nach einem der vorhergehenden Ansprüche, **dadurch gekennzeichnet, dass** eine digitale Farbkamera vom Typ TRI-CCD oder ein digitaler Fotoapparat vom Typ D70S verwendet wird.

8. Verfahren nach Anspruch 8, **dadurch gekennzeichnet, dass** der Bildaufnahmebereich jedwedem für die Gesamtalterung des Gesichts oder des Körpers repräsentativen Hautbereich entspricht, vorteilhafterweise ist der Bereich aus der Gruppe bestehend aus einem Außenbereich des Auges (Krähenfüße), einem mittleren Bereich der Stirn, einem Bereich der Wange und einem Bereich der Nasolabialfalte ausgewählt.

9. Verfahren zur Bewertung der Anti-Aging-Wirkung eines Kosmetikprodukts, **dadurch gekennzeichnet, dass** das Verfahren zur Charakterisierung von Hautunregelmäßigkeiten an jeder Person einer repräsentativen Gruppe von Personen, wie es in einem der Ansprüche 1 bis 8 definiert ist, verwendet wird und für jede Person:
- wenigstens ein erstes digitales Bild vor der Behandlung mit dem Kosmetikprodukt wenigstens eines bestimmten Hautbereichs aufgenommen wird, um die Referenzparameter der Haut vor der Behandlung zu bestimmen,
- wenigstens ein zweites digitales Bild nach einer vorbestimmten Zeitdauer der Behandlung mit dem Kosmetikprodukt aufgenommen wird und die gleichen Parameter an dem zweiten Bild bestimmt werden,
- die Parameter des nach der Behandlung erhaltenen zweiten Bildes des gleichen Hautbereichs mit den Parametern des ersten Hautbildes vor der Behandlung verglichen werden,
- jeder Parameter über die Gesamtheit der Personen der Gruppe gemittelt wird,
- dann, wenn wenigstens einer der betrachteten Durchschnittsparameter, ausgewählt aus der Varianz der Graustufen, sich gegenüber dem Mittelwert der Varianz der Graustufen des ersten Bildes signifikant verringert hat, auf die positive Wirksamkeit des Anti-Aging-Effektes des betrachteten Kosmetikprodukts geschlossen wird, und
- diese Anti-Aging-Wirkung an einem Panel von Personen einer für eine Kategorie gegebenen Alters repräsentativen Gruppe realisiert wird, um das signifikante Anti-Aging-Wirksamkeitsmerkmal des betrachteten Kosmetikproduktes zu bestimmen.

10. Verfahren nach Anspruch 9, **dadurch gekennzeichnet, dass** die signifikante Verringerung mit einer Fehlerwahrscheinlichkeit von weniger als oder gleich 5 % erreicht wird.

11. Verfahren nach einem der Ansprüche 9 bis 10, **dadurch gekennzeichnet, dass** das erste Bild und das zweite Bild an einem jeden der unterschiedlichen, für die Alterung der Haut im Gesicht und/oder am Körper charakteristischen Bereiche, insbesondere:
- einem Außenbereich des Auges (Krähenfüße),
- einem mittleren Bereich der Stirn,
- einem Bereich der Wange und
- einem Bereich der Nasolabialfalte
aufgezeichnet werden.

12. Gerät (10) zur Charakterisierung von mit der Alterung zusammenhängenden Hautunregelmäßigkeiten einer Person, umfassend:
a) eine Digitalkamera oder einen digitalen Fotoapparat (12), die bzw. der ermöglicht, wenigstens ein digitales Bild (14) wenigstens eines bestimmten Hautbereichs (34, 36, 38, 40), wobei das Bild durch eine Vielzahl von Pixeln definiert ist, aufzunehmen, das an eine Vorrichtung zur Verarbeitung digitaler Bilder übertragen wird,
**dadurch gekennzeichnet, dass** es umfasst:
b) Mittel zum Aufzeichnen einer Vielzahl von Bildern der Haut einer gleichen Person, an mehreren unterschiedlichen Bereichen, auf einer Vorrichtung zur Speicherung digitaler Daten,
c) Mittel, um aus dem digitalen Bild eine begrenzte Fläche der zu analysierenden Haut der Person zu extrahieren, an der Berechnungsmittel die Analyse der Hautunregelmäßigkeiten über diese gesamte begrenzte Fläche durchführen, dann Mittel zum Zerlegen des digitalen Bildes in drei Farbebenen: Rot (60), Grün (70), Blau (80), bezeichnet mit R, G, B, mit Hilfe der Bildverarbeitungsvorrichtung,
d) Mittel zum Extrahieren einer einzigen dieser Ebenen, die durch die blaue Farbebene (80) gebildet ist, für die Betrachtung von Falten, von Fältchen und einer Pigmentstörung,
e) Mittel zum Berechnen von wenigstens einem der folgenden Parameter anhand dieser Ebene, gebildet durch die Varianz der Graustufen über alle Pixel des Bildes, als für die Homogenität der Haut repräsentativer Parameter, und die Berechnungsmittel berücksichtigen den Mittelwert eines jeden Parameters, der an den unterschiedlichen Bildern erhalten wird, die jeweils mit den betrachteten unterschiedlichen Bereichen des Gesichts erhalten werden, um jeden durchschnittlichen Gesamtparameter des Gesichts zu erhalten, der gespeichert wird und für jede Person verwendet wird, um eine Eichkurve des Alters der Haut zu bilden.

13. Gerät nach Anspruch 12, **dadurch gekennzeichnet, dass** die Extraktionsmittel das Extrahieren der für die zu betrachtende Unregelmäßigkeit repräsentativen Farbebene vollziehen, auf der die Berechnungsmittel die vorgenannte Berechnung wenigstens eines Parameters, ausgewählt aus der mittleren Graustufe des Bildes, der Oberfläche oder Fläche der Hautunregelmäßigkeiten, von denen einige mit der Alterung zusammenhängen, durchführen.

14. Gerät nach Anspruch 12 oder 13, **dadurch gekennzeichnet, dass** es ferner eine Vorrichtung zur Beleuchtung mit weißem Licht oder UV-Licht umfasst, die die Aufnahme wenigstens eines Bildes wenigstens eines Bereichs der Haut unter einer solchen Beleuchtung ermöglicht.

15. Gerät nach einem der Ansprüche 12 bis 14, **dadurch gekennzeichnet, dass** es Mittel zur Berücksichtigung eines Graustufen-Schwellwertvergleichs umfasst, das heißt Mittel, die ermöglichen, die Graustufen unterhalb eines gewissen vorbestimmten Graustufen-Schwellwerts zu eliminieren, um Störelemente zu beseitigen.

16. Gerät nach einem der Ansprüche 15 bis 18, **dadurch gekennzeichnet, dass** die begrenzte Fläche der Haut eine Fläche von etwa 1 cm² bis 10 cm² ausmacht.

17. Gerät nach einem der Ansprüche 13 bis 16, **dadurch gekennzeichnet, dass** es Mittel zum Vergrößern des mittels der Kamera (12) erhaltenen Bildes umfasst, die einer Bedienungsperson ermöglichen, die Hautunregelmäßigkeiten besser sichtbar zu machen und den Graustufen-Schwellwertvergleich, der ermöglicht, die Störelemente oder Artefakte zu beseitigen, auszuwerten.

18. Gerät nach einem der Ansprüche 12 bis 17, **dadurch gekennzeichnet, dass** eine digitale Farbkamera (12) vom Typ TRI-CCD oder ein digitaler Fotoapparat vom Typ D70S vorgesehen ist.

19. Gerät nach einem der Ansprüche 12 bis 18, **dadurch gekennzeichnet, dass** der Bildaufnahmebereich jedwedem für die Gesamtalterung des Gesichts oder des Körpers repräsentativen Hautbereich entspricht, vorteilhafterweise ist der Bereich aus der Gruppe bestehend aus einem Außenbereich des Auges oder Krähenfüßen, einem mittleren Bereich der Stirn, einem Bereich der Wange und einem Bereich der Nasolabialfalte ausgewählt.

20. Gerät nach einem der Ansprüche 12 bis 19, **dadurch gekennzeichnet, dass** es einen Computer (50) umfasst, der mit einem einen Bildschirm (54) aufweisenden Monitor (52), einer Tastatur (56) und einer Maus (58) kombiniert ist und der eine Software umfasst, die alle vorgenannten Mittel integriert, einschließlich:
- der Mittel zum Zerlegen des digitalen Bildes in drei Farbebenen: Rot (60), Grün (70), Blau (80), bezeichnet mit R, G, B,
- der Mittel zum Extrahieren einer einzigen dieser Ebenen (80), gebildet durch die sogenannte blaue Ebene, die der Farbe Blau entspricht,
- der Mittel zum Berechnen von wenigstens einem der folgenden Parameter anhand dieser Ebene:
- der mittleren Graustufe des Bildes,
- der Oberfläche oder Fläche der Hautunregelmäßigkeiten des Bildes, von denen einige mit der Alterung zusammenhängen, wie Falten, Fältchen, Flecken, Augenringe, Pigmentstörungen, Erschlaffungsbereiche,
- der Mittel zur Berücksichtigung eines Graustufen-Schwellwertvergleichs,
- der Mittel zur Vergrößerung des Bildes,
- der Mittel zur Aufzeichnung wenigstens eines Bildes oder einer Vielzahl von Bildern der Haut.

## Claims

1. Method of characterizing the imperfections of the skin of a person linked to ageing, comprising the following step:
a) taking of at least one digital image (14) of at least one determined skin zone (34, 36, 38, 40), the said image being defined by a multiplicity of pixels with the aid of a digital video camera or digital still photo or picture camera (12), that is transmitted to a digital image processing device; **characterized in that** it comprises the following steps:
b) selecting a limited area of the skin of the person to be analysed is chosen, on which the analysis of the skin imperfections is undertaken on the entirety of this area, recording a plurality of images of the skin of one and the same person over several different zones, on a device for recording digital data, and then splitting the thus recorded digital images into three colour planes: red (60), green (70), blue (80), termed R, G, B, with the aid of the said image processing device;
c) extracting a single of these planes (80) constituted by the blue color plane (80) for the observation of wrinkles, sign lines and pigmentary defects;
d) calculating at least one of the following parameters, by appropriate means of calculation, on the basis of this plane constituted by the variance of the grey levels over the whole set of pixels of the image as parameter representative of the homogeneity of the skin, and the calculation means take into account the mean of each parameter obtained over the different images obtained respectively with the different zones of the face that are considered, to obtain each global mean parameter of the face which is recorded and used, by each person to constitute a calibration curve for the age of the skin.

2. Method according to Claim 1, **characterized in that**, on the selected colour plane, carring out said parametrization of at least one parameter chosen from among the mean grey level of the image, the surface area of the skin imperfections, some of which are related to aging.

3. Method according to Claim 1 or 2, **characterized in that**,
said image taking of at least one skin zone is performed under lightening with a white light or with an ultraviolet light.

4. Method according to one of Claims 1 to 3, **characterized in** carrying out a thresholding of the grey levels, that is to say eliminating the grey levels below a certain predetermined grey level threshold, so as to eliminate the spurious elements.

5. Method according to to one of Claims 1 to 5, **characterized in that** the said limited area represents an area of about from 1 cm² to 10 cm².

6. Method according to one of Claims 1 to 5, **characterized in that** an enlargement of the said image obtained by the video camera or still photo or picture camera is performed allowing an operator to better view the skin imperfections and to evaluate the thresholding of the grey levels making it possible to eliminate the spurious elements or artefacts.

7. Method according to one of the preceding claims, **characterized in** using a digital video colour camera of TRI-CCD type, or a digital still photo or picture camera of type D70S.

8. Method according to claim 8, **characterized in that** the said image-taking zone corresponds to any cutaneous zone representative of the global ageing of the face or of the body; advantageously, said zone is selected from the group consisting of an outer zone of the eye (crow's-foot), of a middle zone of the brow; of a zone of the cheek, and of a zone of the nasogenian furrow.

9. Method of assessing the anti-aging effect of a cosmetic product, **characterized in that** the method of characterizing the imperfections of the skin is used on each person of a representative group of people, such as defined in any one of Claims 1 to 8; and for each person:
- taking of at least one first digital image before treatment with the said cosmetic product of at least one determined skin zone to determine the reference parameters of the skin before treatment;
- taking of at least one second digital image after a predetermined time period of the said treatment with the cosmetic product and the same parameters are determined on the said second image;
- comparing the parameters of the second image of the same skin zone obtained after treatment with respect to the parameters of the first image of skin before treatment;
- determining the means of each parameter over the set of people of the group; and
- reaching a conclusion of the positive effectiveness of the anti-ageing effect of the cosmetic product considered when at least one of the mean parameters considered chosen from the variance of the grey levels, has decreased significantly with respect to the mean of the variance of the grey levels of the first image.

10. Method according to Claim 9, **characterized in that** the significant decrease is obtained with a probability of error of less than or equal to 5%.

11. Method according to any one of Claims 9 to 10, **characterized in that** the first image and the second image are recorded on each of the different characteristic zones of the ageing of the skin on the face and/or the body, in particular from:
- an outer zone of the eye (crow's-foot),
- a middle zone of the brow;
- a zone of the cheek, and
- a zone of the nasogenian furrow.

12. Apparatus (10) for characterizing the imperfections of the skin of a person, comprising:
a) a digital video camera (12) or a digital still photo or picture camera (12) allowing the taking of at least one digital image (14) of at least one determined skin zone (34, 36, 38, 40), the said image being defined by a multiplicity of pixels, that is transmitted to a digital image processing device; **characterized in that** it comprises:
b) means for recording a plurality of images of the skin of one and the same person over several different zones, on a device for recording digital data ;
c) means for extracting from the digital image a limited area of the skin of the person to be analysed, on which the analysis of the skin imperfections is undertaken on the entirety of this area, and then splitting the thus recorded digital images into three colour planes: red (60), green (70), blue (80), termed R, G, B, with the aid of the said image processing device;
d) means for extracting a single of these planes (80) constituted by the blue color plane (80) for the observation of wrinkles, sign lines and pigmentary defects;
e) calculation means for calculating at least one of the following parameters, from this plane, constituted by the variance of the grey levels over the whole set of pixels of the image as parameter representative of the homogeneity of the skin, and the calculation means take into account the mean of each parameter obtained over the different images obtained respectively with the different zones of the face that are considered, to obtain each global mean parameter of the face which is recorded and used, by each person to constitute a calibration curve for the age of the skin.

13. Apparatus according to Claim 12, **characterized in that** the extraction means carry out the extraction of the colour plane representative of the imperfection to be observed, on which the calculation means carry out the aforesaid calculation of at least one parameter chosen from among the mean grey level of the image, the surface area of the skin imperfections, some of which are related to aging.

14. Apparatus according to Claim 12 or 13, **characterized in that** it further comprises a lightening device for lightening with a white light or with an ultraviolet light enabling the taking of at least one image of at least one skin zone under such a lightening.

15. Apparatus according to one of Claims 12 to 14, **characterized in that** it comprises means of consideration of a thresholding of the grey levels, that is to say means making it possible to eliminate the grey levels below a certain predetermined grey level threshold, so as to eliminate the spurious elements.

16. Apparatus according to one of Claims 15 to 18, **characterized in that** the said limited area of the skin represents an area of about 1cm² to 10 cm².

17. Apparatus according to one of Claims 12 to 16, **characterized in that** it comprises means of enlargement of the said image obtained by the camera (12) allowing an operator to better view the skin imperfections and to evaluate the thresholding of the grey levels making it possible to eliminate the spurious elements or artefacts.

18. Apparatus according to one of Claims 12 to 17, **characterized in that** a digital colour video camera (12) of TRI-CCD type, or a digital still photo or picture camera of type D70S, is provided.

19. Apparatus according to one of Claims 12 to 18, **characterized in that** said image taking zone corresponds to any cutaneous zone representative of a global aging of the face or of the body; advantageously, said zone is selected from the group consisting of an outer zone of the eye (crow's-foot), a middle zone of the brow; a zone of the cheek, and a zone of the nasogenian furrow.

20. Apparatus according to one of Claims 12 to 19, **characterized in that** it comprises a computer (50) combined with a monitor (52) comprising a screen (54), a keyboard (56) and a mouse (58) and comprising software integrating the whole of the aforesaid means including:
- the means of splitting the digital image into three colour planes: red (60), green (70), blue (80), termed R, G, B;
- the means of extracting a single of these planes (80) constituted by the plane termed blue corresponding to the colour blue;
- the means of calculating at least one of the following parameters from this plane:
- the mean grey level of the image;
- the surface area of the imperfections of the skin of the said image, some of which are related to aging, such as wrinkles, sign lines, spots, dark circles, pigmentary imperfections, slackening or loosening zones;
- the means of consideration of a thresholding of the grey levels;
- the means of enlargement of the said image;
- the means of recording at least one image or a plurality of images of the skin.
